# EUROPEAN PATENT SPECIFICATION

(11) **EP 4 208 237 B1**
(45) Date of publication and mention of the grant of the patent: **06.11.2024**
(21) Application number: 21769661.6
(22) Date of filing: 20.08.2021
(51) Int. Cl.: A61M 16/00, A61M 15/00, A61M 11/00, A61M 16/06

(54) **AEROSOL HIGH FLOW THERAPY APPARATUS**
AEROSOL-THERAPIEVORRICHTUNG MIT HOHEM DURCHFLUSS
APPAREIL THÉRAPEUTIQUE À DÉBIT ÉLEVÉ D'AÉROSOL

(30) Priority: 01.09.2020 EP 20193976
(43) Date of publication of application: 12.07.2023
(60) Divisional application: 24202870.2
(73) Proprietor: Stamford Devices Limited, H91 HE94 Galway (IE)
(72) Inventor: POWER, John, Dangan Galway, H91 HE94 (IE); DUFFY, Aidan, Dangan Galway, H91 HE94 (IE); CASEY, Micheal, Dangan Galway, H91 HE94 (IE)
(74) Representative: Weldon O'Brien Ltd.
(86) International application number: PCT/EP2021/073209
(87) International publication number: WO 2022/048927

(56) References cited:
- EP-A2- 0 018 805
- WO-A1-2005/089845
- WO-A1-2007/058574
- WO-A1-2017/004404
- WO-A1-2019/115802
- WO-A2-2005/035021
- WO-A2-2005/118036
- WO-A2-2008/116165
- US-A- 4 248 218
- US-A- 4 328 797
- US-A- 5 871 011
- US-A- 6 135 109
- US-A1- 2003 168 063
- US-A1- 2006 191 537
- US-A1- 2010 122 705
- US-A1- 2012 285 457
- US-A1- 2014 251 333
- US-A1- 2017 182 275
- US-A1- 2017 259 018
- US-A1- 2018 064 898
- US-A1- 2019 099 581
- US-B1- 6 736 140
- US-B1- 9 186 474

## Description

### Introduction

The present invention relates to aerosol therapy, especially high flow therapy.

High flow nasal therapy (HFNT) will typically deliver an air/oxygen/aerosol mix to a patient at a rate that exceeds their peak inspiratory rate. An example is 50LPM treatment vs. an average inhalation of about 20 LPM (averaged across the inhalation period of a breath) with a peak inhalation of about 35LPM. Aerosol delivered into the high flow stream will be homogeneously distributed. Therefore, the excess airflow contains aerosolised drug that the patient cannot absorb and this results in reduced efficiency. Also, this excess will disperse into the surrounding room air. This is a fugitive emission that potentially exposes clinicians, patients and visitors to aerosolised drugs and patient-generated pathogens.

Also, during exhalation (typically through the mouth), the high flow therapy may continue to deliver to the nasal cavity. A portion of this flow will travel into the cavity and exit the patient's mouth, and this flow augments the exhalation flowrate and has the potential to collect patient pathogens. The remainder of the flow will exit the cavity via the nostrils, but prior to this it also can collect pathogens.

WO2015/155342A (Stamford Devices Ltd) and WO2019/007950A (Stamford Devices Ltd) describe HFNT systems, in which aerosol is delivered primarily during reduced gas flow periods, in order to increase efficiency and reduce losses. US2012/285455 (Varga et al) describes a mask for patient ventilation. US2004/244799 (Landis) describes a tube seal adapter for face masks. WO2018/204969 (P & M Hebbard PTY) describes a sealing pad for a respiratory mask. WO2019/159063 (Fisher & Paykel) describes a mask which is fitted over a nasal prong interface. WO2017/004404 (Vapotherm) Describes a nasal cannula. US2014/0251333 (BURK et al) describes an exhalation scavenging mask. US9,186,474 (Rollins, III) describes a multi-function oxygen mask. WO2005/118036 (Chathampally Y. G.) describes systems and methods for administration of drugs and medications). WO2008/116165 (Next Safety) describes methods and systems of delivering medication via inhalation.

The invention addresses the problem of achieving effective aerosol treatment with reduction or elimination of gas losses, particularly for high flow treatment.

### Summary of the Disclosure

The invention provides a system as set out in claim 1, and optional features as set out in claims 2-14.

### Detailed Description of the Invention

The invention will be more clearly understood from the following description of some embodiments thereof, given by way of example only with reference to the accompanying drawings in which:
Figs. 1 to 4 are perspective views of an aerosol high flow mask being applied in four steps, respectively;
Fig. 5 is a diagram of a mask in position on a demonstration patient head model used for testing;
Fig. 6 is a plot of bench test results showing particle numbers vs. time determined by an aerodynamic particle analyser for the set-up of Fig. 5 and 50LPM High Flow therapy, and
Fig. 7 is a plot of bench test results showing particle measurements with time for various high flow rates, also for the set-up of Fig. 5 and in this case showing in more detail the plots for the higher-level extraction of 70 to 135 LPM;
Fig. 8 is a plot, also for this set-up, showing percentage of particles captured with extraction flow rate;
Fig. 9 shows fluid flows detected by imaging sensors, coded to illustrate volumetric velocity within the mask for nominal mask fit, lower mask fit, and closed mask fit;
Fig. 10 is a set of plots of extraction flowrate with time for a set-up as shown in Fig. 5 and with a nasal pressure sensor added;
Fig. 11 is a histogram of peak nasal pressure for extraction being turned on and off, and
Fig. 12 is a histogram of minimum nasal pressure for extraction turned on and off;
Fig. 13 is a diagram showing the mask of Figs. 1 to 4 in place, together with a controller;
Fig. 14 is a flow diagram showing how reduced flow is achieved during inhalation and the aerosol chamber limb is bypassed during inhalation;
Fig. 15 is a pair of plots showing dynamic extraction and pressure monitoring, showing relationship between extraction and breath flows, in which extraction is matched to aerosol flow; and
Fig. 16 is a diagram showing flows of humidified and heated air and O₂ towards the mask and extracted humid breath which is dried in the extraction direction by virtue of a heat recovery condenser and a heat exchanger which recovers heat for humidification.

### Description of the Embodiments

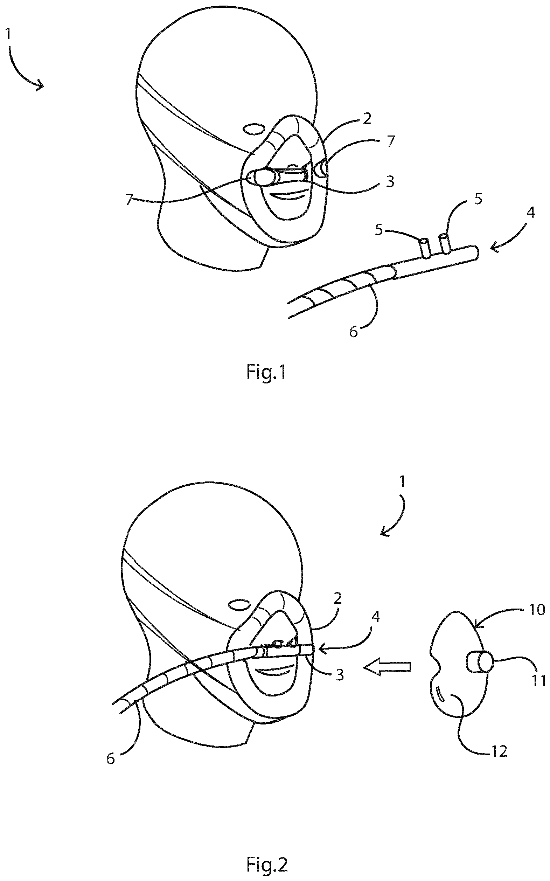

Referring to Fig. 1 a patient interface 1 comprises an annular soft seal base 2 configured to fit around a patient's mouth and nose with a large contact surface of resilient material which is suited to skin contact. The base 2 has a relatively rigid core surrounded on at least the patient side with soft material for patient facial contact. The base spine supports a prong receiver 3 extending across the base 2, and being of a plastics material having a rugged strength to support attachment of a nasal prong head 4 with prongs 5 at the end of tubing 6 for delivery of high flow gas and aerosol to the nostrils.

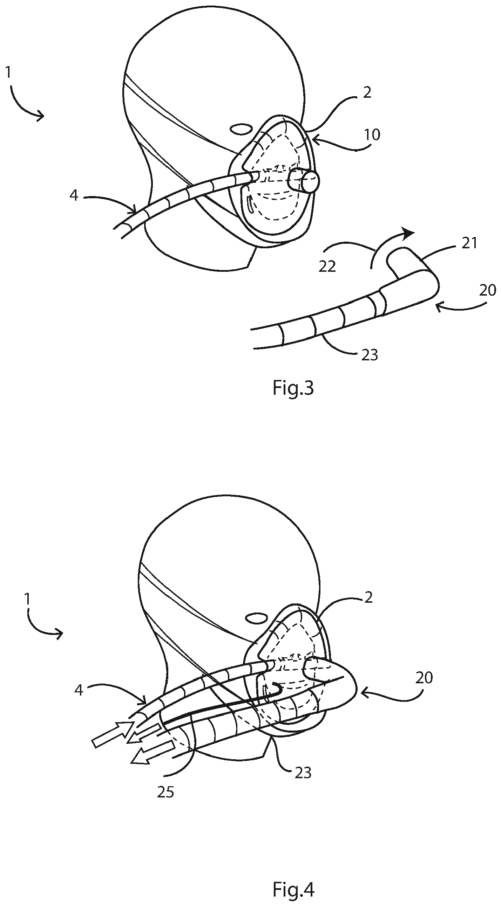

Fig. 2 shows that the interface also includes a shell 10 which fits with a seal to the base 2 and the support 3 to form an enclosed volume around the patient's mouth and nasal area. Fig. 3 shows an extraction head 20 which has an L-shaped conduit 21 with a flange 22 configured to fit to a cylindrical port 11 of the shell.

Fig. 4 shows the full interface 1, with all of these components in place, together with a lead 25 from a pressure sensor within the volume formed by the base 2 and the shell 10. The pressure sensor is attached to the inside surface of the shell 10.

The patient interface is modular, the soft seal base 2 attaching to the patient's head with securing head straps 8. The support 3 supports the prong head 4 with the prongs 5 correctly aligned. It is envisaged that in other embodiments the prong support is self-supporting by way of head straps rather than being attached to the soft seal base, especially for uses without extraction.

The soft seal base 2 is placed on the face first and a comfortable sealing surface is established. This establishes a gas-tight seal and provides a support mechanism for the high flow therapy tubing, and the clinician can conveniently and accurately set up the patient's nasal prongs 3 secured to the soft seal base 2. The fit of the prongs 5 can be checked and adjusted.

The shell 10 can now be assembled. The perimeter of the shell 10 interfaces with the base such that it self-locates and forms a seal, the soft seal base 2 providing a means of securing the shell 10 in place by for example elastic straps, hook-and-loop fasteners, or clips. It is preferred that the shell fit by way of a snap-fit connection with resilient edges. The shell 10, nasal prongs 4/5, and the soft seal base 2 are profiled such that the high flow therapy tubing and head 4 can enter from either the left or the right, and a seal is still established without the need for an additional part. In another embodiment an additional capping feature can be provided.

The shell 10 preferably includes one or more vents to prevent an excessive negative pressure drop within the volume formed by the base 2 and the shell 10 due to extraction. These are located away from exhalation / exhaust airstreams of the mouth and the nostrils. The shell 10 has a port 11 to attach a means of suction, in this case the extraction head 20. The position of the extraction port 11 is such that in use it is opposed to the mouth and nostrils for optimal collection of exhaled / exhausted gasses and particles. The vent or vents may not be in the mask itself, and could for example be part of an exhalation tube. The benefit of a vent is that, because the mask is very effective at sealing the space around the nose and mouth, the operation of the high flow system and the forced extraction system does not cause the system to be too intrusive by acting effectively akin to a ventilator, in which all inhalation and exhalation is controlled. There may for example be a very soft opening on an inhale valve to not affect breathing, and/or a pressure release valve for safely in case of reduced extraction. The vent may have a suitable filter to block outflow of unwanted droplets to avoid contamination of the environment.

The shell also has a retainer to attach a sensor for measuring the internal mask pressure.

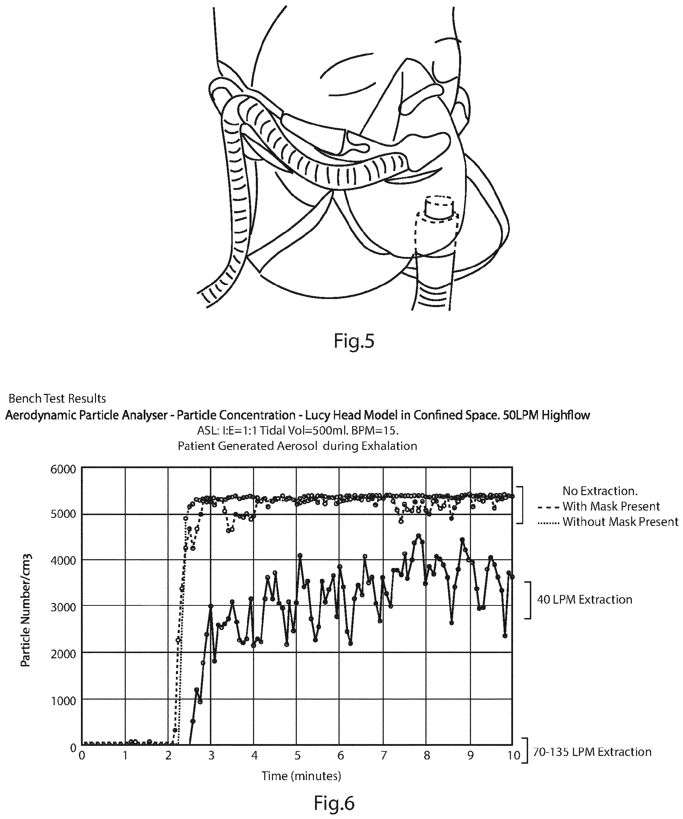

Figs. 6 to 8 shows test results for ambient aerosol particles in the environment surrounding the patient's head for with the mask and system shown in Fig. 5. This mask is conventional in terms of how it is configured to engage the face, simply a flexible clear polymer with holes cut to take the HFNT delivery and extraction hoses. It does not have the benefits of the mask of Figs. 1 to 4.

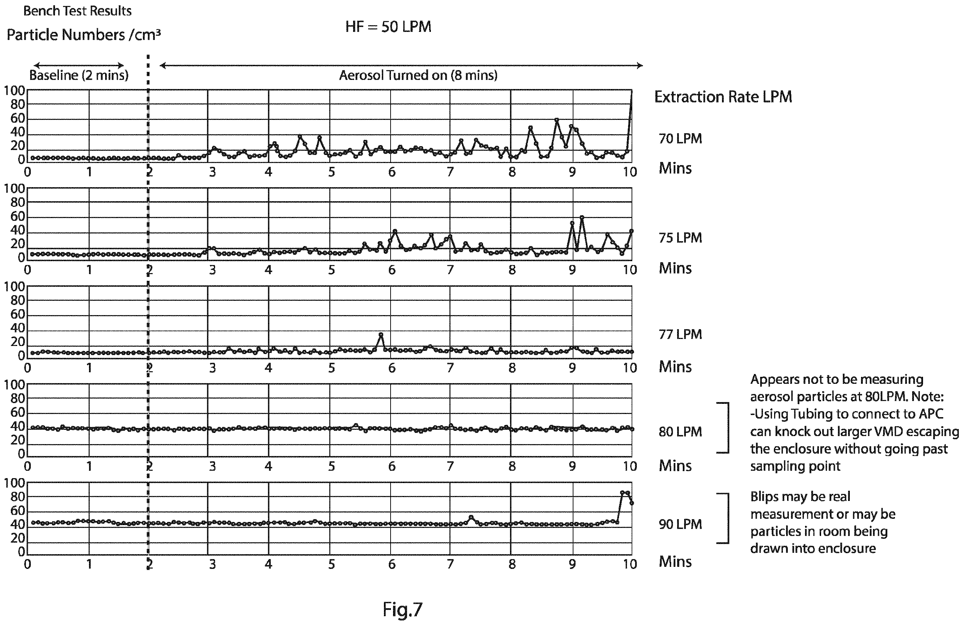

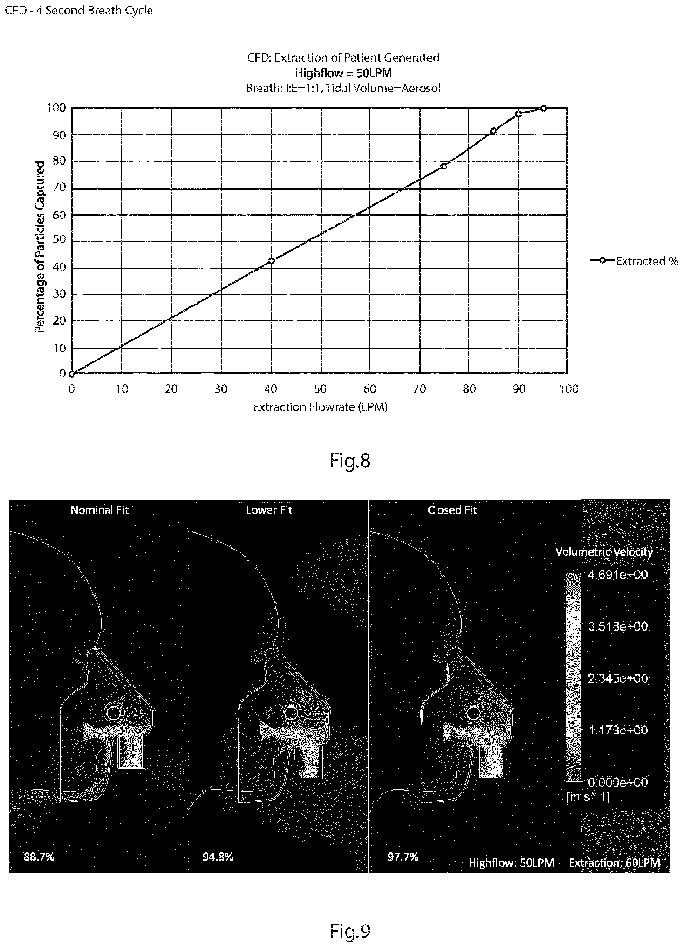

Fig. 6 demonstrates an extraction flowrate in the region of 80LPM to 100LPM is required to capture approximately all aerosol particles, in these examples with treatment at 50LPM. Fig. 6 demonstrates the major differences between the bands of 7- to 135 LPM, 40 LPM, and no extraction. In the latter there is essentially no difference between situations where the mask is present and is not, because of losses around the edges of the mask. Fig. 7 shows more detail for the higher extraction rate band, with the vertical axes showing particles in the tens per cm³. Fig. 9 is a summary histogram showing that there is a liner relationship between extraction flowrate and percentage of particles captured., reaching full capture at about 90LPM extraction.

The positioning of the extraction port opposite the patient's mouth affects the rate of emission capture for a given extraction rate. In Fig. 9, the `Lower Fit' outperforms the `Nominal Fit' in terms of capture of emissions (94.8% vs. 88.7%). The degree of sealing between the mask and the face affects rate of emission capture for a given extraction rate. In Fig. 9 `Closed Fit' is the same as `Lower Fit' except that there is sealing at the cheeks. At the same level of extraction (60 LPM) versus the same emission flowrate (50LPM, there is no breath in this example) the `Closed Fit' outperforms the `Lower Fit' in term of capture of emissions (97.7% vs. 94.8%).

The effect of extraction on the nasal cavity has been investigated. A test setup as shown in Fig. 5 but with addition of a nasal pressure sensor involved:
Delivery of humified high flow @ 50LPM
Pressure tube inserted into nasal cavity
Extraction flowrate either Off or @100LPM
Flowmeter in-line with extraction source.

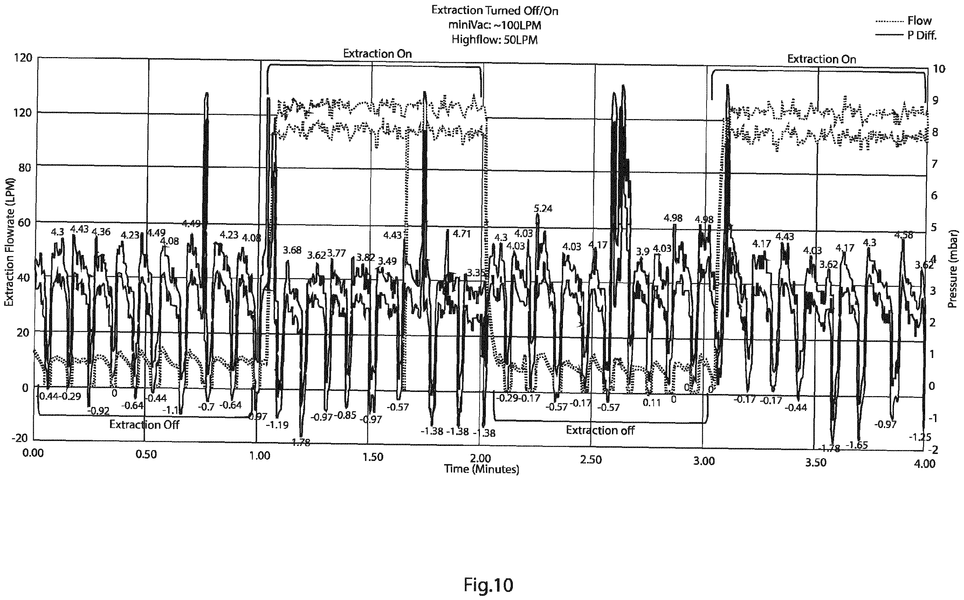

The results of this are illustrated in Figs. 10 to 12, which displays the distribution of the nasal pressure peaks, which occur during exhalation, with and without extraction. The average difference is a reduction of 0.3mBar when extraction is applied; the difference of the maximums is 0.53mBar.

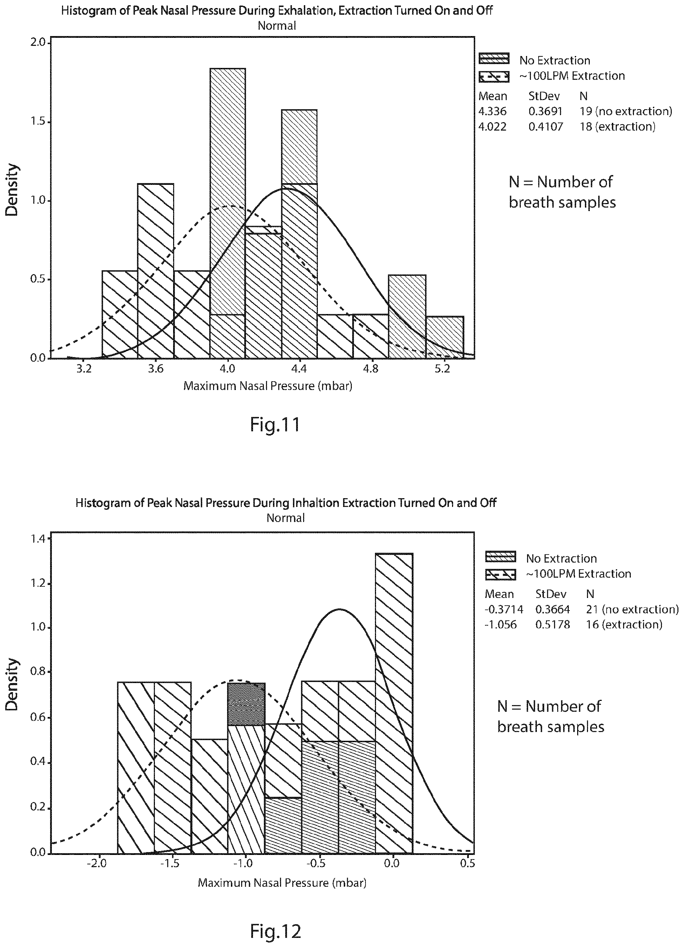

Fig. 12 displays the distribution of the nasal pressure troughs (these occur during inhalation) with and without extraction. The average difference is a reduction of 0.68mBar when extraction is applied; the difference of the minimums is also 0.68mBar.

These results demonstrate the advantages of decreasing extraction during exhalation and not having any extraction during inhalation.

Major advantages of the invention include:
Flexible patient-engaging side of the base 2 provides consistent sealing, especially allowing successful extraction at lower flow rates.
A predictable seal allows less safety factor buffer on the applied extraction rate.
Less extraction allows less pressure drop, less noise, more extraction source options, controlled vent ports.
Maximise extraction for a given extraction flowrate.
Avoidance of occlusion with patient's face.
Reduced wind noise / wind feeling
Sized to alleviate pressure drop by a predictable quantity
Modularity, allowing fitting of the base to be secured to head and adjusted for sealing and comfort independent of the therapy.
Allows clinical access to setup / check/ change prongs.
Pressure Port.
Provides means of measuring pressure internal to the mask.
Can be used as a safety measure.
Can be used to detect the breath and dynamically control the extraction rate.
Allows for more effective extraction with reduced impact on pressure drop.

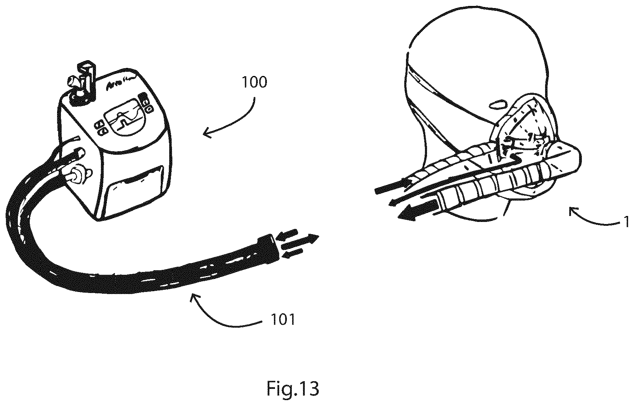

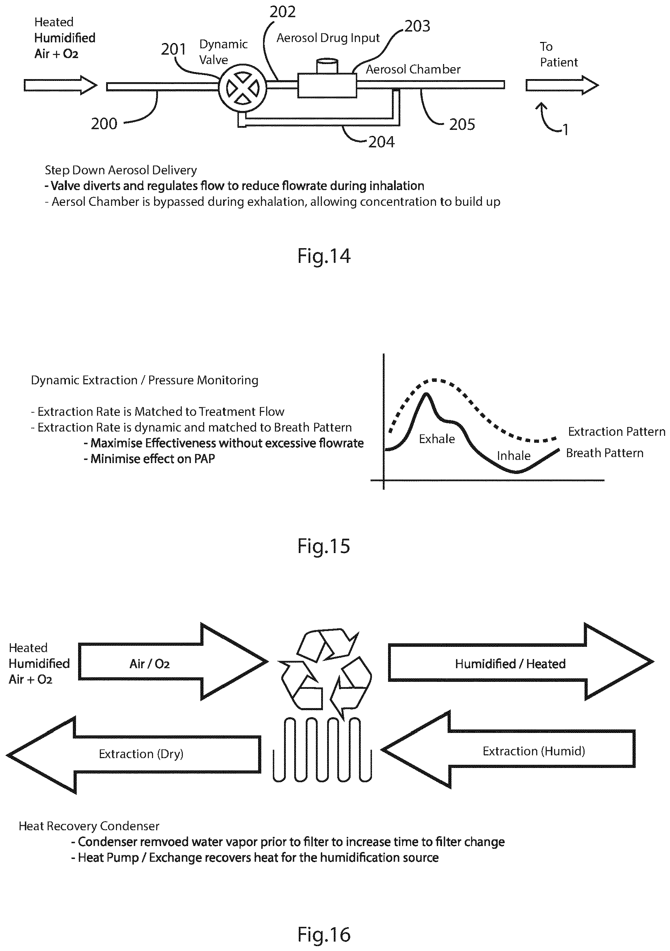

The patient interface 1 can connect to a standalone aerosol / high flow therapy device 100 by a tubing set 101 as depicted in Fig. 13. This allows breath-synchronised step-down aerosol delivery. As illustrated in Fig. 14 a heated humidified air/ O₂ mixture is delivered on a tube 200, and flow is split by a valve 201 into an aerosol branch 202 with a nebulizer 203 and a parallel bypass branch 204. The branches merge into a common tube 205 which leads to the interface 1.

In some examples, the aerosol delivery path can include an aerosol chamber having an increased volume to slow down the flowrate at the point of aerosol delivery

### Bypass Scenarios

The valve 201 can dynamically throttle the flowrate, and can dynamically divert the flowrate to provide scenarios such as:
Full bypass, normal high flow treatment with no aerosol.
No bypass, open throttle, continuous aerosol delivery.
No bypass, open throttle, breath synchronised aerosol delivery (pressure sensor used to detect breath pattern).
Breath-synchronised bypass: divert through bypass during exhalation. Nebuliser is continuously on allowing aerosol to accumulate in the chamber. During inhalation divert the flow through the chamber for increased aerosol concentration.

### Step-Down Delivery

Step-down aerosol delivery: when switching to the aerosol path 202 during inhalation, the average flowrate is reduced. This will improve dose efficiently. The period of reduced flowrate is short to prevent de-recruitment effects. There is a ramp down / ramp up of the reduced flowrate, and these ramps can be controlled / modulated to minimise de-recruitment and discomfort.

### Dynamic Extraction / Pressure Monitoring

The system controller can adapt the baseline extraction to match the high flow therapy setting. The controller can be programmed to dynamically change the extraction rate to match the breath pattern, as illustrated in Fig. 15. This can maximise the effectiveness of extraction, and therefore reduce the required extraction rate. This has the benefit of reducing the impact on the treatment pressure, and also reduces the extraction source requirements.

### Filtration

An advantageous part of the extraction system is that there is filtration in line with the extracted airflow to capture any pathogens or drug before it is vented to the ambient room. This can be a standard commercial filter that can be changed out by the clinicians. Due to the large levels of humidity in the expelled gas, the filter will become saturated, and the filter regime adapted accordingly. The system can determine the actual flowrate based off the mask pressure readings. Or, additional flow and pressure sensors could be employed on the system side of the filter. The system can increase the power supplied to the extraction source to keep the extraction flowrate consistent over time as the filter approaches saturation.

### Condenser

A condenser can be employed to take vapour out of the extracted gas prior to it reaching the filter. This can prolong the life of the filter. The condensing mechanism is preferably such that the surfaces that make contact with the extracted gasses are part of a disposable circuit. A heat pump (for example using a Peltier heat exchanger) can be employed to increase the rate of condensing, as illustrated in Fig. 16. The heat collected in this exchange can be employed in heating of the high flow therapy delivered to the patient. This would have energy saving benefits.
No Fugitive emissions of treatment drug
No spreading of patient pathogens
Increased drug efficiency
Increased drug rate

The invention is not limited to the embodiments described but may be varied in construction and detail within the scope of the claims. For example, it is envisaged that the mask is provided as a pre-assembled component, perhaps using a sizing chart to allow a clinician to preconfigure and position nasal prongs. The mask, if it does not have a removable shell may have an access flap to allow adjustment of the nasal prongs. The performance features and advantages described for an interface having conventional features apply to an interface of Figs. 1 to 4, and it is expected that performance would be better because of the enhanced sealing and other advantages described with reference to Figs. 1 to 4. Irrespective of the interface used, any interface which supports the HFNT delivery and extraction and provides a closed or near-closed environment around the mouth and nose is advantageous as described above with reference to Figs. 5 to 12 and 14 to 16, and where the interface of Figs. 1 to 4 and 13 is used it enhances the closed nature of this volume around the nose and mouth.

## Claims

1. An aerosol treatment system comprising:
a patient interface (1) to cover a patient's mouth and nose,
an aerosol delivery apparatus (4, 6),
a high flow treatment system (200 - 205) linked with the patient interface (1),
a controller (100),
a heater and a humidifier, separately or combined, to provide a heated humidified air/O2 mixture (200) delivered to the patient interface (1), and
sensors for detecting patient breathing,
wherein the controller (100) is configured to control delivery of aerosol to provide breath-synchronised aerosol delivery,
**characterized in that** the system comprises:
a valve (201) arranged to split delivery flow into an aerosol branch (202) with a nebulizer (203) and a parallel bypass branch (204), and the branches merge into a common tube (205) which leads to the patient interface (1).

2. An aerosol treatment system of claim 1, further comprising an extraction apparatus (20), and wherein the controller (100) is configured to control delivery of gas to the patient interface and extraction of gases from a volume enclosed by the patient interface.

3. An aerosol treatment system as claimed in claims 1 or 2, wherein the high flow treatment system is a high flow nasal treatment system (HFNT).

4. An aerosol treatment system as claimed in any of claims 1 to 3, wherein the controller (100) is configured to dynamically control the system to provide scenarios including one or more of:
a full bypass;
no bypass with continuous aerosol delivery;
no bypass with breath synchronised aerosol delivery according to signals from a pressure sensor in the volume enclosed by the interface; and/or
breath-synchronised bypass with divert through the bypass during exhalation; continuously-on aerosol generation allowing aerosol to accumulate in a chamber and during inhalation divert the flow through the chamber for increased aerosol concentration.

5. An aerosol treatment system as claimed in any of claims 1 to 4, wherein the controller (100) is configured to provide step-down aerosol delivery.

6. An aerosol treatment system as claimed in claim 5, wherein the controller (100) is configured to provide reduced gas flowrate and increased aerosol delivery during inhalation to improve dose efficiently.

7. An aerosol treatment system as claimed in claim 6. ^wherein the controller (100) is configured to provide a period of reduced flowrate which is short enough to prevent de-recruitment effects.

8. An aerosol treatment system as claimed in claim 2 or any of claims 3 to 7 when dependent on claim 2, wherein the controller (100) is configured to provide dynamic extraction according to monitoring of pressure in said interface volume.

9. An aerosol treatment system as claimed in claim 8, wherein the controller (100) is configured to adapt a baseline extraction to match a high flow therapy setting, and to dynamically change an extraction rate to match patient's breath pattern.

10. An aerosol treatment system as claimed in claim 2 or any of claims 3 to 9 when dependent on claim 2, wherein the extraction apparatus (20) includes a filter adapted to capture pathogens or drug before venting to ambient

11. An aerosol treatment system as claimed in 10, wherein the controller (100) is configured to increase power supplied to the extraction apparatus (20) to keep the extraction flowrate consistent over time as the filter approaches saturation.

12. An aerosol treatment system as claimed in any of claims 10 or 11, wherein the extraction apparatus (20) comprises a condenser to take vapour out of extracted gas, preferably prior to it reaching the filter.

13. An aerosol treatment system as claimed in claim 12, wherein the condenser is included in a heat pump in which heat collected is used to heat delivery flow to the patient.

14. An aerosol treatment system as claimed in any of claims 1 to 13, wherein the patient interface (1) comprises
a base (2) configured to surround at least part of a patient's mouth and nose and engage the skin with a resilient seal,
a support (3) on the base for supporting an aerosol or gas delivery head,
a shell (10) configured to form an enclosure together with the base, and
an extraction port (11) for attachment of the or an extraction system to extract gas from a volume enclosed by the patient interface in use.

## Patentansprüche

1. Aerosol-Behandlungssystem, das Folgendes umfasst:
ein Patienten-Interface (1), um den Mund und die Nase eines Patienten zu bedecken,
eine Aerosolabgabevorrichtung (4, 6),
ein High-Flow-Behandlungssystem (200-205), das mit dem Patienten-Interface (1) verknüpft ist,
einen Regler (100),
ein Erwärmungselement und einen Befeuchter, getrennt oder kombiniert, um eine erwärmte befeuchtete Luft-O₂-Mischung (200) bereitzustellen, die an das Patienten-Interface (1) abgegeben wird, und
Sensoren zur Detektion der Patientenatmung,
wobei der Regler (100) zur Regelung der Abgabe von Aerosol konfiguriert ist, um eine mit Atmung abgestimmte Aerosolabgabe bereitzustellen,
**dadurch gekennzeichnet, dass** das System Folgendes umfasst:
ein Ventil (201), das zur Teilung des Abgabeflusses in eine Aerosolabzweigung (202) angeordnet ist, mit
einem Zerstäuber (203) und einer parallelen Bypass-Abzweigung (204), und die Abzweigungen zu einer gemeinsamen Röhre (205) zusammenführen, die zu dem Patienten-Interface (1) führt.

2. Aerosol-Behandlungssystem nach Anspruch 1, das weiter eine Extraktionsvorrichtung (20) umfasst und wobei der Regler (100) zur Regelung der Abgabe von Gas an das Patienten-Interface und Extraktion von Gasen aus einem Volumen, das von dem Patienten-Interface umschlossen ist, konfiguriert ist.

3. Aerosol-Behandlungssystem nach den Ansprüchen 1 oder 2, wobei das High-Flow-Behandlungssystem ein High-Flow-Nasenbehandlungssystem (HFNT) ist.

4. Aerosol-Behandlungssystem nach einem der Ansprüche 1 bis 3, wobei der Regler (100) zur dynamischen Regelung des Systems konfiguriert ist, um Szenarien bereitzustellen, die eines oder mehrere von folgenden umfassen:
einen vollständigen Bypass;
keinen Bypass mit kontinuierlicher Aerosolabgabe;
keinen Bypass, mit Atmung abgestimmte Aerosolabgabe entsprechend Signalen von einem Drucksensor in dem Volumen, das von dem Interface umschlossen ist; und/oder mit Atmung abgestimmter Bypass mit Umleitung durch den Bypass während der Ausatmung;
kontinuierliche Aerosolerzeugung, was ermöglicht, dass sich Aerosol in einer Kammer ansammelt und während der Einatmung der Fluss durch die Kammer für eine erhöhte Aerosolkonzentration umgeleitet wird.

5. Aerosol-Behandlungssystem nach einem der Ansprüche 1 bis 4, wobei der Regler (100) zur Bereitstellung von Step-Down-Aerosolabgabe konfiguriert ist.

6. Aerosol-Behandlungssystem nach Anspruch 5, wobei der Regler (100) zur Bereitstellung von reduzierter Gasflussrate und erhöhter Aerosolabgabe während der Einatmung konfiguriert ist, um die Dosis effizient zu verbessern.

7. Aerosol-Behandlungssystem nach Anspruch 6, wobei der Regler (100) zur Bereitstellung einer Dauer von reduzierter Flussrate konfiguriert ist, die kurz genug ist, um Derecruitment-Effekte zu verhindern.

8. Aerosol-Behandlungssystem nach Anspruch 2 oder einem der Ansprüche 3 bis 7, bei Abhängigkeit von Anspruch 2, wobei der Regler (100) zur Bereitstellung von dynamischer Extraktion entsprechend der Überwachung von Druck in dem Interface-Volumen konfiguriert ist.

9. Aerosol-Behandlungssystem nach Anspruch 8, wobei der Regler (100) für Folgendes konfiguriert ist: zur Anpassung einer Grundlinienextraktion, um einer High-Flow-Therapieeinstellung zu entsprechen, und zur dynamischen Änderung einer Extraktionsrate, um einem Atmungsmuster des Patienten zu entsprechen.

10. Aerosol-Behandlungssystem nach Anspruch 2 oder einem der Ansprüche 3 bis 9, bei Abhängigkeit von Anspruch 2, wobei die Extraktionsvorrichtung (20) einen Filter einschließt, der zum Einfangen von Pathogenen oder Arzneimitteln vor der Entlüftung an die Umgebung geeignet ist.

11. Aerosol-Behandlungssystem nach Anspruch 10, wobei der Regler (100) zur Erhöhung der Kraft konfiguriert ist, die der Extraktionsvorrichtung (20) zugeführt wird, um die Extraktionsflussrate über die Zeit konstant zu halten, wenn sich der Filter einer Sättigung nähert.

12. Aerosol-Behandlungssystem nach einem der Ansprüche 10 oder 11, wobei die Extraktionsvorrichtung (20) einen Kühler umfasst, um Dampf aus extrahiertem Gas zu entfernen, bevorzugt bevor es den Filter erreicht.

13. Aerosol-Behandlungssystem nach Anspruch 12, wobei der Kühler in einer Wärmepumpe eingeschlossen ist, in der gewonnene Wärme zur Erwärmung des Abgabeflusses an den Patienten verwendet wird.

14. Aerosol-Behandlungssystem nach einem der Ansprüche 1 bis 13, wobei das Patienten-Interface (1) Folgendes umfasst:
ein Unterteil (2), das zur Umschließung von zumindest einem Teil des Munds und der Nase eines Patienten und zum Eingreifen in die Haut mit einer elastischen Dichtung konfiguriert ist,
eine Halterung (3) auf dem Unterteil zum Halten eines Aerosol- oder Gasabgabekopfes, eine Außenschale (10), die, zusammen mit dem Unterteil, zur Bildung eines Gehäuses konfiguriert ist, und
einen Extraktionsanschluss (11) zur Anbringung des oder eines Extraktionssystems, um Gas aus einem Volumen, das von dem Patienten-Interface umschlossen ist, bei Verwendung zu extrahieren.

## Revendications

1. Système de traitement par aérosol comprenant :
une interface de patient (1) pour couvrir la bouche et le nez d'un patient,
un appareil de délivrance d'aérosol (4, 6),
un système de traitement à haut débit (200 - 205) lié à l'interface de patient (1),
un dispositif de commande (100),
un élément de chauffage et un humidificateur, séparément ou combinés, pour fournir un mélange humidifié chauffé d'air/O2 (200) délivré à l'interface de patient (1), et
des capteurs destinés à détecter la respiration du patient,
dans lequel le dispositif de commande (100) est configuré pour commander la délivrance d'aérosol pour fournir une délivrance d'aérosol synchronisée avec la respiration,
**caractérisé en ce que** le système comprend :
une vanne (201) agencée pour séparer l'écoulement de délivrance en une branche d'aérosol (202) avec un nébuliseur (203) et une branche de dérivation parallèle (204), et les branches fusionnent en un tube commun (205) qui mène à l'interface de patient (1).

2. Système de traitement par aérosol selon la revendication 1, comprenant en outre un appareil d'extraction (20), et dans lequel le dispositif de commande (100) est configuré pour commander la délivrance de gaz à l'interface de patient et l'extraction de gaz d'un volume entouré par l'interface patient.

3. Système de traitement par aérosol selon les revendications 1 ou 2, dans lequel le système de traitement à haut débit est un système de traitement nasal à haut débit (HFNT).

4. Système de traitement par aérosol selon l'une quelconque des revendications 1 à 3, dans lequel le dispositif de commande (100) est configuré pour commander de manière dynamique le système pour fournir des scénarios incluant un ou plusieurs des suivants :
une dérivation totale ;
aucune dérivation avec une délivrance d'aérosol continue ;
aucune dérivation avec une délivrance d'aérosol synchronisée avec la respiration selon des signaux d'un capteur de pression dans le volume entouré par l'interface ; et/ou
une dérivation synchronisée avec la respiration qui dévie à travers la dérivation pendant l'exhalation ;
la génération d'aérosol en continu permettant que l'aérosol s'accumule dans une chambre et pendant l'inhalation dévie l'écoulement à travers la chambre pour une concentration d'aérosol augmentée.

5. Système de traitement par aérosol selon l'une quelconque des revendications 1 à 4, dans lequel le dispositif de commande (100) est configuré pour fournir une délivrance d'aérosol réduite.

6. Système de traitement par aérosol selon la revendication 5, dans lequel le dispositif de commande (100) est configuré pour fournir un débit de gaz réduit et une délivrance d'aérosol augmentée pendant l'inhalation pour améliorer l'efficacité de la dose.

7. Système de traitement par aérosol selon la revendication 6, dans lequel le dispositif de commande (100) est configuré pour fournir une période de débit réduit qui est suffisamment courte pour empêcher les effets de dérecrutement.

8. Système de traitement par aérosol selon la revendication 2 ou l'une quelconque des revendications 3 à 7 quand elles dépendent de la revendication 2, dans lequel le dispositif de commande (100) est configuré pour fournir une extraction dynamique selon la surveillance de la pression dans ledit volume d'interface.

9. Système de traitement par aérosol selon la revendication 8, dans lequel le dispositif de commande (100) est configuré pour adapter une extraction de ligne de base pour correspondre à un réglage de thérapie à haut débit, et pour changer de manière dynamique un taux d'extraction pour correspondre au schéma de respiration du patient.

10. Système de traitement par aérosol selon la revendication 2 ou l'une quelconque des revendications 3 à 9 quand elles dépendent de la revendication 2, dans lequel l'appareil d'extraction (20) inclut un filtre conçu pour capturer des pathogènes ou un médicament avant une mise à l'air libre ambiant.

11. Système de traitement par aérosol selon la revendication 10, dans lequel le dispositif de commande (100) est configuré pour augmenter la puissance alimentant l'appareil d'extraction (20) pour garder le débit d'extraction cohérent dans le temps alors que le filtre s'approche de la saturation.

12. Système de traitement par aérosol selon l'une quelconque des revendications 10 ou 11, dans lequel l'appareil d'extraction (20) comprend un condensateur pour faire sortir la vapeur du gaz extrait, de préférence avant qu'elle n'atteigne le filtre.

13. Système de traitement par aérosol selon la revendication 12, dans lequel le condensateur est inclus dans une pompe à chaleur dans laquelle la chaleur collectée est utilisée pour chauffer un écoulement de délivrance de chaleur au patient.

14. Système de traitement par aérosol selon l'une quelconque des revendications 1 à 13, dans lequel l'interface de patient (1) comprend :
une base (2) configurée pour entourer au moins une partie de la bouche et du nez d'un patient et entrer en prise avec la peau avec un joint étanche élastique,
un support (3) sur la base destiné à supporter une tête de délivrance d'aérosol ou de gaz,
une coque (10) configurée pour former une enceinte conjointement avec la base, et
un orifice d'extraction (11) destiné à attacher le ou un système d'extraction pour extraire du gaz d'un volume enfermé par l'interface de patient pendant l'utilisation.
